# EUROPÄISCHE PATENTSCHRIFT

(11) **EP 0 387 921 B1**
(45) Veröffentlichungstag und Bekanntmachung des Hinweises auf die Patenterteilung: **23.08.1995**
(21) Anmeldenummer: 90108063.0
(22) Anmeldetag: 07.05.1987
(51) Int. Cl.: G21K 1/04, A61B 6/06

(54) **Konturenkollimator für die Strahlentherapie**
Radiation therapy contour collimator
Collimateur de contours pour thérapie par irradiation

(30) Priorität: 14.05.1986 DE 3616141; 03.04.1987 DE 3711245
(43) Veröffentlichungstag der Anmeldung: 19.09.1990
(62) Teilanmeldung aus: 87106581.9
(73) Patentinhaber: Deutsches Krebsforschungszentrum Stiftung des öffentlichen Rechts, 69120 Heidelberg (DE)
(72) Erfinder: Pastyr, Otto, D-6906 Leimen (DE); Maier-Borst, Wolfgang, Dr., D-6901 Dossenheim (DE)
(74) Vertreter: Müller-Boré & Partner Patentanwälte

(56) Entgegenhaltungen:
- DE-A- 3 030 332
- DE-B- 1 010 659
- DE-C- 192 300
- US-A- 4 463 266

## Beschreibung

Die Erfindung bezieht sich auf einen Konturenkollimator für die Strahlentherapie mit einer vorgegebenen Anzahl von gegeneinander verschieblich angeordneten Blendplatten. Sie bezieht sich insbesondere auf einen Vielblattkollimator, der zur Begrenzung des Strahlungsfeldes einer ionisierenden Strahlung eingesetzt wird, und zwar bevorzugt zur Begrenzung des Strahlungsfeldes von Gammastrahlung bei einem Linearbeschleuniger.

Die heute in der onkologischen Strahlentherapie eingesetzten Bestrahlungsgeräte sind mit Strahlenfeldkollimatoren ausgerüstet, die lediglich die Einstellung rechteckig begrenzter Strahlenfelder zulassen. Es ist heute jedoch bekannt, daß bei vielen onkologischen Fragestellungen bessere Therapieergebnisse erreicht werden können, wenn die Strahlendosisverteilung der meist unregelmäßigen Form der Zielvolumina (Tumore sind meist nicht kugelförmig) angepaßt wird.

Hierzu werden in der Strahlentherapie unregelmäßig geformte Zusatzkollimatoren individuell angefertig. Die zur Herstellung solcher Kollimatoren erforderlichen Geräte sind kommerziell verfügbar. Es handelt sich dabei um Geräte, die es ermöglichen, anhand von Röntgenbildvorlagen irreguläre Strahlenfeldformen aus Hartschaumplatten auszuschneiden und diese mit Metallegierungen niedrigen Schmelzpunktes auszugießen. Diese Herstellungsprozedur ist nur für einzelne Einstrahlungswinkel durchführbar und recht aufwendig.

Aus der DE-B-1 010 659 ist ein Kollimator zur Ausblendung eines Nutzstrahlenbündels aus der Strahlung eines Strahlers hoher Energie, z. B. eines Kobalt-60-Präparates, bekannt, der Blendplatten aufweist, welche senkrecht zum Zentralstrahl des auszublendenden Bündels verstellbar sind. Bei diesem Kollimator ist je ein Verstellglied für jede einzelne Blendplatte vorgesehen. Ein allen Verstellgliedern gemeinsames Antriebsorgan, z. B. eine Antriebswelle, ist mit jedem der Verstellglieder lediglich über Reibkupplungen verbunden. Die Begrenzung des gewünschten Strahlungsfeldes ist durch eine Lochplatte vorgegeben, in die Stifte hineingesteckt werden. Bei einem solchen Kollimator ist es ersichtlich schwierig, innerhalb kurzer Zeit ein neues Bestrahlungsfeld einzustellen. Darüber hinaus ist der Kollimator für Pendelungen in einer Vertikalebene, die z.B. bei der Therapie mit einem Linearbeschleuniger durchgeführt werden, nicht geeignet. Bei einer gewissen Stellung spricht nämlich unter der Wirkung des Gewichts der Kollimatorplatten die Rutschkupplung an; damit würden Blendplatten herausfallen, wodurch sich eine Verstellung der Kontur ergibt. Darüber hinaus würde eine Rutschkupplung nicht die Patientensicherheit bei der sogenannten Einzeit-Bestrahlung, bei der die gesamte erforderliche Dosis in einer Fraktion abgegeben wird, gewährleisten.

Aus der DE-A-30 30 332 ist eine Primärstrahlenblende für ein Röntgenuntersuchungsgerät bekannt, bei dem mehrere, den Strahlenkegel von verschiedenen Seiten begrenzende Einblendelemente aus paketweise zusammengefaßten dünnen, aneinanderliegenden, in Längsrichtung gegeneinander verschiebbaren Metallstreifen verwendet werden. Zur Einstellung durch Fernsteuerung trägt jeder Metallstreifen an seiner der Symmetrieachse der Primärstrahlenblende abgewandten Seite eine sich quer zur Verschieberichtung und senkrecht zur Einblendebene erstreckende Nase. Darüber hinaus ist jedem Metallstreifenpaket ein mit den einzelnen Metallstreifen in Eingriff bringbares, von einem x-, y-Antrieb verstellbares Verstellglied zugeordnet. Auch dieser Konturenkollimator ist für geringe Energien vorgesehen, da relativ kurze Blendplatten verwendet werden. Bei einer 360°-Drehung des Kollimators um einen Patienten würden die einzelnen Blendplatten herausfallen, weil keine Verriegelung vorgesehen ist. Zwar ist ein Einstellorgan für die einzelnen Blendplatten vorhanden, aber dies kann nur geringe Blendplattengewichte bewegen. Darüber hinaus ist es infolge seiner Konstruktion darauf beschränkt, nur weichgeformte Konturen oder Profile, also solche ohne Stufen, einzustellen.

Aus der US-A-4,463,266 ist ein Konturenkollimator mit keilförmigen Blendplatten bekannt, die so schwenkbar sind, daß die Innenkante der Blendplatten stets auf den Fokus der Strahlungsquelle gerichtet ist. Bei dieser Anordnung ergibt sich jedoch die Schwierigkeit, daß aufgrund der Keilform der Blendplatten bei einem Zurückfahren derselben leicht ein Klemmen auftritt. Für die Blendplatten muß man einerseits ein strahlungsresistentes und strahlungsundurchlässiges Material verwenden, das aber aus Kostengründen mit den üblichen Arbeitsmethoden zu bearbeiten sein soll. Beispielsweise soll es möglich sein, in den Blendplatten ohne allzu großen Aufwand Führungsrillen aufzubringen, oder die Blendplatten keilförmig zu schleifen. Ein Material, das den genannten Anforderungen gerecht wird, ist beispielsweise eine Wolfram-Nickel-Legierung. Eine solche Legierung ist eine relativ hartes, nicht elastisches Material. Werden Blendplatten aus einem solchen Material, also weitgehend starre Platten, von der Mittellinie nach außen verschoben (bei feststehenden Außenplatten), d.h. aus dem Zentrum der Strahlung heraus, dann können aus geometrischen Gründen die Blendplatten klemmen, was ein Zurückfahren schwierig macht. Von Bedeutung ist dieses Problem dann, wenn - wie bei der genannten US-A-4,463,266 - die Blendplatten in Richtung auf den Ursprungsort der Strahlung verjüngen.

Aufgabe der Erfindung ist es somit, den Konturenkollimator derart weiterzubilden, daß auch bei Einsatz von Blendplatten aus relativ hartem Material beim Verschieben kein Klemmen auftritt.

Diese Aufgabe wird dadurch gelöst, daß die Blendplatten zwischen zwei Außenplatten angeordnet und mit einer Kante von zwei Seiten an eine Mittelinie heranschiebbar sind, durch die eine Symmetrielinie der Strahlung verläuft, zur Bildung einer Strahlenkontur 24, wobei die Innenflächen der Außenplatten unter vorgegebenem Winkel zur Normalen der Mittellinie ausgerichtet sind, wobei die Blendplatten der der Mittellinie der Strahlung zugewandten Kante eine geringere Dicke aufweisen als der von der Mittellinie der Strahlung abgewandten Kante und daß die Blendplatten an der der Strahlungsquelle zugewandten Kante eine kleinere Dicke aufweisen als der von der Strahlungsquelle abgewandten Kante.

Vorteilhafte Ausführungsformen sind in den Unteransprüchen gekennzeichnet.

Im folgenden werden Ausführungsbeispiele der Erfindung anhand von zwölf Figuren näher erläutert. Es zeigen:
- Fig. 1: einen Linearbeschleuniger, bei dem ein Konturenkollimator nach der Erfindung eingesetzt wird;
- Fig. 2: einen seitlichen Blick auf einen Konturenkollimator nach der Erfindung;
- Fig. 3: einen Blick auf eine Blendplatte, die im rechten Blendplattenpaket nach Fig. 2 eingesetzt wird;
- Fig. 4: einen Blick auf die das Strahlungsbündel definierende gerade Kante der Blendplatte nach Fig. 3;
- Fig. 5: einen Blick (Ansicht A) auf den Konturenkollimator nach Fig. 2, teilweise aufgebrochen;
- Fig. 6: einen Blick (Ansicht B) auf den linken Teil des Konturenkollimators nach Fig. 2 ohne Blendplatten;
- Fig. 7: einen Schnitt (Schnitt C-C) durch den rechten Teil des Konturenkollimators nach Fig. 2;
- Fig. 8: einen Blick von oben auf einen Konturenkollimator mit keilförmigen Außenplatten entsprechend Fig. 5;
- Fig. 9: einen Blick auf eine Blendplatte, die in der rechten vorderen Blendplatten-Paketgruppe nach Fig. 8 eingesetzt wird;
- Fig. 10: einen Schnitt durch den linken Teil der Blendplatte nach Fig. 9 entlang der Linie X-X;
- Fig. 11: einen Schnitt durch den rechten Teil der Blendplatte nach Fig. 9 entlang der Linie XI-XI; und
- Fig. 12: einen Blick von oben auf die Blendplatte nach Fig. 9.

Im folgenden werden für gleiche Bauteile dieselben Bezugszeichen verwendet.

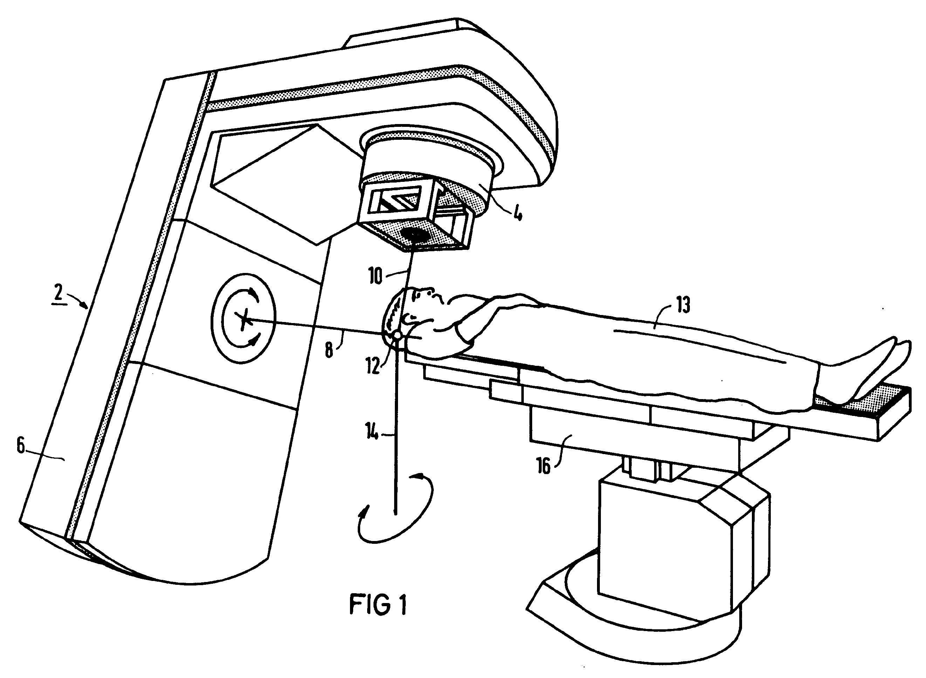

In Fig. 1 ist ein Teil eines Linearbeschleunigers 2 bekannter Ausführung dargestellt, bei dem ein Konturenkollimator 4 mit serieller Ansteuerung der einzelnen Blendplatten eingesetzt ist. Der Linearbeschleuniger 2 umfaßt eine Gantry (Gerüst) 6, die den Konturenkollimator 4 enthält und die um eine horizontale Rotationsachse 8 im Laufe einer therapeutischen Behandlung gedreht wird. Der Hauptstrahl des aus dem Linearbeschleuniger 2 austretenden Strahlungsbündels ist mit 10 bezeichnet. Er ist während der Behandlung auf die zu behandelnde Zone 12 eines Patienten 13 gerichtet, die im Isozentrum liegt. Die Rotationsachse 8 der Gantry 6, die Rotationsachse 14 eines Behandlungstisches 16 und die Strahlachse 10 schneiden sich im Isozentrum.

Die folgende Beschreibung bezieht sich auf einen motorisch verstellbaren Konturenkollimator. Es ist jedoch ohne weiteres erkennbar, daß unter Weglassung aller für den motorischen Antrieb vorgesehener Elemente auch eine manuelle Verstellung möglich ist.

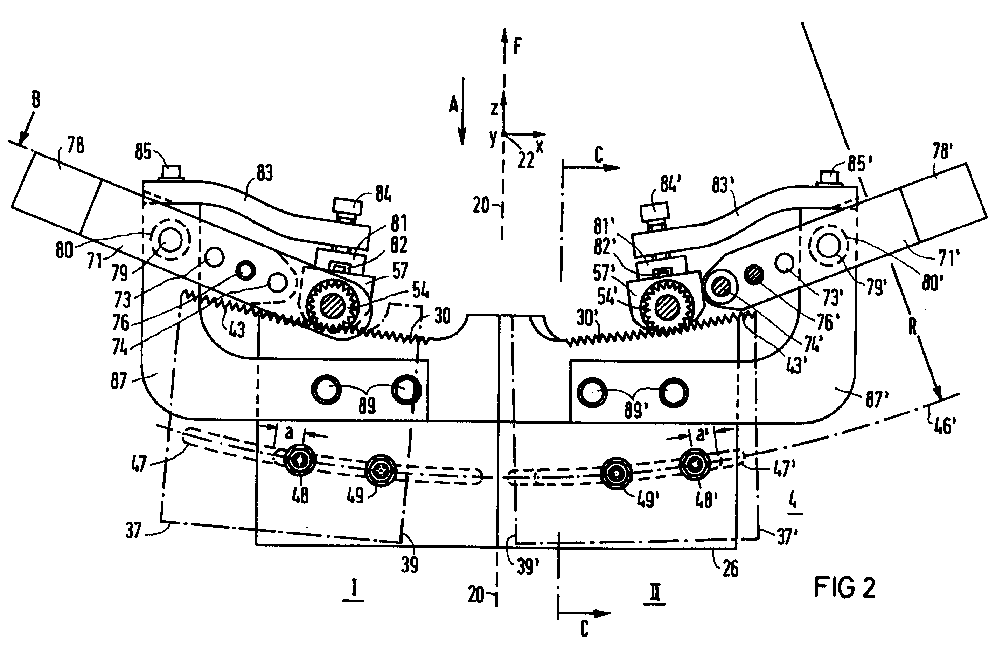

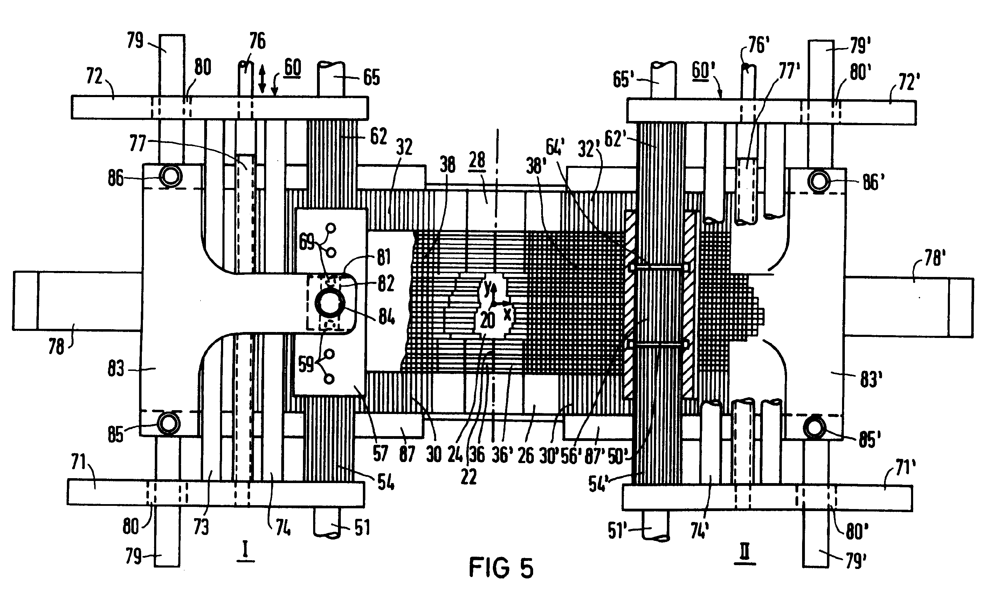

In Fig. 2 sind in einer seitlichen Darstellung Einzelheiten des Konturenkollimators 4 gezeigt; für die Beschreibung ist die vertikale Bestrahlungsstellung angenommen. Dieser Kollimator 4 besteht aus zwei Teilen oder Seiten I und II, die bezüglich einer vertikalen Ebene, durch die eine Symmetrielinie 20 verläuft, symmetrisch zueinander ausgebildet sind. Die Symmetrielinie 20 fällt bei optimaler Justierung mit der Hauptstrahlrichtung des von einem Fokus F ausgehenden Strahlungsbündels hochenergetischer Strahlung zusammen. Bei dieser Strahlung kann es sich insbesondere um Röntgenstrahlung handeln. Wie aus Fig. 5 (Blick in Richtung A in Fig. 2) hervorgeht, liegt zwischen den Seiten I und II eine Mittellinie 22, die zusammen mit der Symmetrielinie 20 die vertikale Symmetrieebene 20, 22 aufspannt. Die Mittellinie 22 fällt mit der y-Achse eines x-, y-, z-Koordinatensystems zusammen. Dessen z-Achse wird durch die Symmetrielinie 20 gebildet. Die im Bereich der Symmetrielinie 20 liegende, durch Ausblendung erzielte Strahlenkontur (Profil) ist mit 24 bezeichnet.

Aus einer Betrachtung der Fig. 2 und 5 ergibt sich, daß in einigem Abstand voneinander eine erste und eine zweite vertikal gestellte, seitliche Außenplatte 26 bzw. 28 parallel zueinander angeordnet sind. Diese Außenplatten 26, 28 sind an ihren Oberkanten ab einem gewissen Abstand zur Symmetrieebene 20, 22 in kreisbogenförmigen Ausnehmungen oder Rändern mit Verzahnungen 30, 30′ bzw. 32, 32′ versehen.

Die Seiten I und II sind bezüglich der Symmetrieebene 20, 22 symmetrisch aufgebaut, so daß es genügt, lediglich die linke Seite I im einzelnen zu beschreiben. Die entsprechenden, auf der rechten Seite II gelegenen Bauelemente sind jeweils mit einem Strich an der zugeordneten Bezugsziffer versehen. Sie haben den gleichen Aufbau und diesselbe Funktion. Auf sie wird im folgenden auch gelegentlich eingegangen.

Zwischen den beiden seitlichen Außenplatten 26, 28 ist ein Paket aus zwei Gruppen von gegeneinander verschieblichen Blendplatten 36, 38 angeordnet. Alle Blendplatten 36, 38 in der vorderen bzw. hinteren Gruppe sind in gleicher Weise aufgebaut und nebeneinander liegend angeordnet. Sie sind dennoch mit verschiedenen Bezugszeichen belegt, weil sie - wie im folgenden näher geschildert wird - durch verschiedene Einrichtungen betätigt werden. Das gesamte Paket kann beispielsweise 28 Blendplatten 36, 38 umfassen. Dasselbe gilt für die Blendplatten 36′, 38′ des rechts angeordneten Blendplattenpakets.

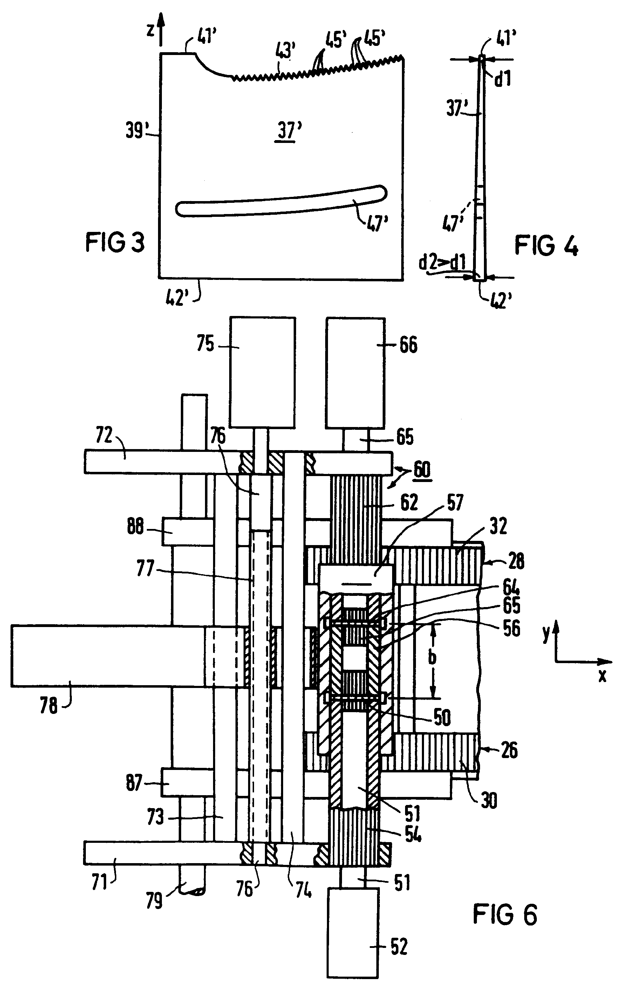

In den Fig. 3 und 4 ist eine beleibige der identischen Blendplatten 36′, 38′ der rechten Seite II näher dargestellt und mit 37′ bezeichnet. Sie ist im wesentlichen quadratisch geformt. Die links gelegene gerade Kante 39′ dient zur Begrenzung des Strahlungsbündels. Die obere Kante 41′ ist mit einer kreisbogenförmigen Ausnehmung (Rand) versehen, in die rechts eine Verzahnung 43′ eingelassen ist. Diese Verzahnung 43′ besitzt vorzugsweise dreieckförmige Zähne 45′, die in einem Abstand von jeweils etwa 1,5 mm angeordnet sind. Infolge dieser feinen Verzahnung 43′ können die Blendplatten 36′, 38′ individuell und feinstufig etwa parallel zur x-z-Ebene geschwenkt werden, und zwar jeweils in Schritten von 1,5 mm. Die Verzahnung 43′ stimmt mit den rechten Verzahnungen 30′, 32′ der festgehaltenen Außenplatten 26, 28 überein.

Unterhalb ihrer Mitte ist in die Blendplatte 37′ eine bogenförmige Führungsrille 47′ eingelassen. Diese gekrümmte Führungsrille 47′ dient zur Führung der Blendplatte 37′ derart, daß die gerade Kante 39′ immer parallel zum äußeren Strahl des eingegrenzten Strahlenkegels verläuft. Mit anderen Worten, während der Führung entlang der Führungsrille 47′ ist die gerade Kante 39′ immer auf den Fokus F der Strahlungsquelle ausgerichtet. Der Krümmungsradius R für diese Führungsrille 47′ kann beispielsweise 53 cm betragen. Der Krümmungsradius R ist in Fig. 2 am Schwenkbogen 46′ eingezeichnet. Die Blendplatte 37′ kann insbesondere aus Wolfram oder einem Wolfram enthaltenden Material bestehen. Nach Fig. 4 besitzt sie einen keilförmigen Querschnitt. Mit anderen Worten, die der Strahlungsquelle und damit dem Fokus F zugewandte Kante 41′ besitzt eine Dicke dl, die kleiner ist als die Dicke d2 an der der Strahlungsquelle abgewandten Kante 42′.

Die Blendplatten 36, 38 der linken Seite I sind identisch ausgebildet zu den Blendplatten 36′, 38′. Sie sind nur seitenverkehrt zwischen den Außenplatten 26, 28 angeordnet. Generell werden sie jeweils als Blendplatte 37 bezeichnet.

In Fig. 2 ist je eine Blendplatte 37, 37′ aus dem Paket der linken bzw. rechten Seite I bzw. II gestrichelt eingezeichnet. Beide Blendplatten 37, 37′ sind in einer von der Symmetrieebene 20, 22 herausgeschobenen Position gezeigt. Sie sind gegenüber den Außenplatten 26, 28 verschoben.In Fig. 2 ist auch zu sehen, daß die beiden Blendplatten 37, 37′ in dieser Position um den Fokus F herumgeschwenkt sind. Zum Führen entlang der Führungsrillen 47, 47′ beim Schwenken (Antrieb über die Verzahnungen 43, 43′) dienen Führungsbolzen 48, 49 bzw. 48′, 49′, die die seitlichen Außenplatten 26, 28 miteinander verbinden. Wenn der Konturenkollimator 4 in einer symmetrischen Stellung und ganz geschlossen ist, dann liegen die geraden Kanten 39, 39′, die auf den Fokus F ausgerichtet sind, in der Symmetrieebene 20, 22. Damit aber die Blendplatte 37 auch in den Bereich der rechten Seite II hineinfahren kann (und entsprechend die Blendplatte 37′ in den Bereich der linken Seite I), sind die Führungsrillen 47, 47′ etwas länger ausgeführt als für die genannte symmetrische Schließstellung eigentlich erforderlich. Dies ist durch den Abstand a bzw. a′ in Fig. 2 angedeutet. Es hat sich gezeigt, daß man mit einem Abstand a = a′ = 10 mm bei einer Blendplatte 37, 37′ von einer Basisbreite von 10 cm ausreichend weit in die benachbarte Seite II bzw. I hineinfahren kann.

Für die vorgegebene Anzahl von Blendplatten 36 der vorderen Gruppe ist ein gemeinsames Verstellorgan vorgesehen. Dieses dient zum seriellen Verschieben jeweils einer ausgewählten ersten dieser Blendplatten 36 gegenüber den restlichen Blendplatten 36. Wie weiter unten erläutert ist, steht dieses Verstellorgan mit der Verzahnung 43 der ausgewählten Blendplatte 36 im Eingriff. Weiterhin ist eine mit den Verzahnungen 43 der restlichen Blendplatten 36 der vorderen Gruppe im Eingriff stehende Verriegelungseinrichtung vorgesehen. Darüber hinaus existiert auch eine Einrichtung zum Verschieben des Verstellorgans von der Verzahnung der ausgewählten Blendplatte zur Verzahnung einer benachbarten zweiten Blendplatte. Wenn das Verstellorgan von der ersten zur zweiten Blendplatte 36 verschoben wird, wird durch letztere Einrichtung die erste Blendplatte 36 verriegelt und die benachbarte zweite Blendplatte 36 entriegelt. Nun kann auch die zweite Blendplatte 36 gegenüber allen nunmehr arretierten Blendplatten 36 verschoben (verschwenkt) werden. Stattdessen kann auch zu einer dritten, vierten, etc. Blendplatte 36 übergegangen und diese verschwenkt werden.

Ein entsprechendes Verstellorgan und entsprechende Einrichtungen sind auch für die hintere Gruppe der Blendplatten 38 vorgesehen. Die beiden Einrichtungen zum Verschieben der Verstellorgane sind weitgehend identisch, d. h. durch diesselben Bauelemente gebildet.

Zunächst werden das Verstellorgan und die Verriegelungseinrichtung für die vordere Gruppe der Blendplatten 36 betrachtet; später wird dann zu der hinteren Gruppe übergegangen.

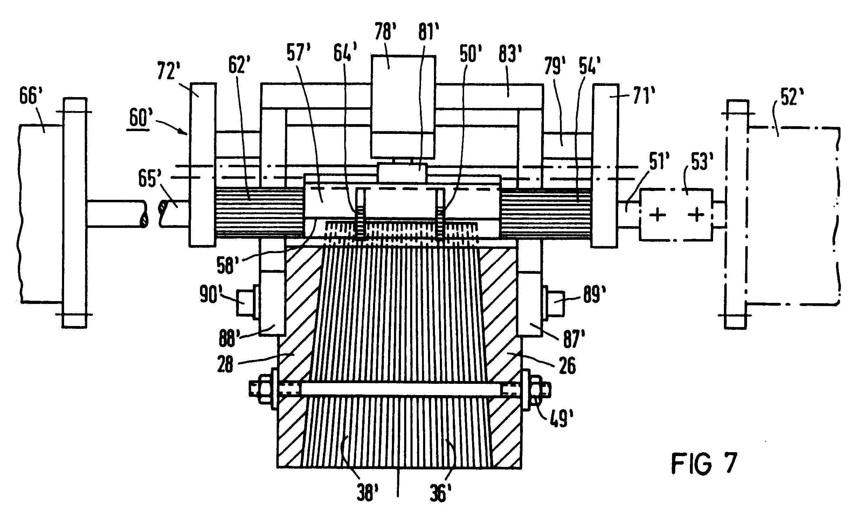

Nach Fig. 5 und 6 umfaßt dieses Verstellorgan ein Antriebszahnrad 50, das etwa die an der Verzahnung 43 gemessene Dicke d1 der zugeordneten Blendplatten 36 besitzt und mit der ausgewählten Blendplatte 36 im Eingriff steht. Dieses Zahnrad 50 ist über eine Einstellwelle 51 mit einem elektrischen Motor 52, insbesondere mit einem Schrittmotor, verbunden. Die Einstellwelle 51 reicht bis etwa zur Mitte des Systems, vergleiche Fig. 6. Wie aus Fig. 7 hervorgeht, kann zwischen dem vorderen Ende der Einstellwelle 51 und dem Motor 52 noch eine Kupplung 53 angeordnet sein, vgl. dort die Kupplung 53′. Der hintere Endbereich der Einstellwelle 51 ist verzahnt (vgl. Fig. 6) ausgebildet, und das Antriebszahnrad 50 ist auf diesen Endbereich aufgeschoben. Dreht sich der Motor 52, so dreht sich auch das Antriebszahnrad 50 in der gewünschen Richtung, wobei die ausgewählte Blendplatte 36 über ihre Verzahnung 43 mitgenommen wird, bis sie die gewünschte Endposition eingenommen hat.

Zu dem Verstellorgan 50, 51, 52 für die ausgewählte Blendplatte 36 gehört auch eine Verriegelungseinrichtung. Diese umfaßt eine erste und eine zweite verzahnte Welle 54 bzw. 56. Diese beiden Wellen 54, 56 sind breite Zahnräder mit Längsbohrungen, die axial zueinander ausgerichtet sind. Die Verzahnung ist so gewählt, daß sie mit den Verzahnungen 30, 32 der Seitenplatten 26, 28 und den Verzahnungen 43 der Blendplatten 36, 38 übereinstimmt. Das Antriebszahnrad 50 liegt axial zwischen den beiden Wellen 54 und 56. Sein Außendurchmesser sowie seine Verzahnung sind identisch mit dem Außendurchmesser bzw. der Verzahnung der Wellen 54, 56. Die Einstellwelle 51 ist dabei durch die Längsbohrung der Welle 54 hindurch- und in die Längsbohrung der Welle 56 hineingeführt. Die beiden Wellen 54, 56 sind durch ein hülsenförmiges Verbindungsteil 57 mit unterer Ausnehmung 58 (vgl. Fig. 7, Ausnehmung 58′) zum Ermöglichen eines Eingriffs in die Verzahnungen 43 miteinander verbunden. Die hierfür verwendeten, oben angeordneten Befestigungsschrauben sind in Fig. 5 mit 59 bezeichnet. Die beiden Walzen oder Wellen 54, 56 sind Teil eines Rahmengestells 60 und somit nicht um ihre Längsachsen drehbar. Von dem gesamten Abschnitt zwischen dem vorderen Ende der Welle 54 und dem hinteren Ende der Welle 56 ist also nur derjenige Teil um die Längsachse drehbar, der durch das Antriebszahnrad 50 eingenommen wird. Die beiden Wellen 54, 56 dienen somit zur Arretierung der darunterliegenden Blendplatten 36, während das Antriebszahnrad 50 zum Schwenken, d. h. zur Verschiebung der ausgewählten Blendplatte 36 in x-Richtung, vorgesehen ist.

Aus Fig. 5 und 6 ist ersichtlich, daß noch eine dritte verzahnte Welle 62 gleichen Durchmessers und gleicher Verzahnung vorgesehen ist. Diese ist axial zu den beiden anderen Wellen 54, 56 ausgerichtet und hat dieselbe Breite wie die Welle 54. Sie dient mit der Welle 56 als Arretierorgan für die nicht ausgewählten Blendplatten 38 der hinteren Gruppe. Entsprechend ist zwischen den beiden Wellen 56 und 62 ein zweites Antriebszahnrad 64 gleichen Durchmessers angeordnet. Dieses sitzt auf dem einen Endbereich einer zweiten Einstellwelle 65, die dort ebenfalls eine Verzahnung aufweist. Die zweite Einstellwelle 65 ist am anderen Ende ebenfalls mit einem Motor 66 verbunden. Zwischen der zweiten Einstellwelle 65 und dem Motor 66 kann auch hier wieder eine (nicht gezeigte) Kupplung vorgesehen sein. Der Motor 66 ist zum Schwenken, d. h. zum Vorschub, für die jeweils ausgewählte hintere Blendplatte 38 parallel zur x-Richtung vorgesehen. Das motorische Verschwenken erfolgt solange, bis die betreffende Blendplatte 38 die gewünschte Endposition eingenommen hat. Die Bauteile 64, 65, 66 bilden somit gemeinsam ein weiteres Verstellorgan.

Durch Verwendung zweier Verstellorgane 52, 51, 50 sowie 66, 65, 64 ist gewährleistet, daß die Einstellzeit, in der die Blendplatten 36, 38 in der Verschieberichtung x eingestellt werden, halbiert wird.

Der Abstand b zwischen den beiden Antriebszahnrädern 50, 64 sollte bei kleinen zu bestrahlenden Feldern relativ klein gewählt werden. Dann können beide Antriebseinheiten zum Einsatz kommen. Das hülsenförmige Verbindungsstück 57 hält auch die dritte Welle 62 axial zu den beiden übrigen Wellen 54, 56 ausgerichtet. Zur Befestigung sind auch hier oben Schrauben 69 vorgesehen. Diese sind mit den Schrauben 59 in einer Linie parallel zur y-Achse angeordnet. Aus Fig. 6 ist ersichtlich, daß sämtliche drei Wellen 54, 56 und 62 Bestandteil des zuvor erwähnten Rahmengestells 60 sind.

Dieses Rahmengestell 60 ist Bestandteil der bereits erwähnten Einrichtung zum Verschieben des genannten Verstellorgans. Es umfaßt einen ersten und einen zweiten Seitenarm 71 bzw. 72, die parallel zur x-Achse ausgerichtet sind und etwa im mittleren Bereich durch zwei parallele Führungsstangen 73 bzw. 74 starr miteinander verbunden sind. Die beiden Führungsstangen 73, 74 sind dabei endseitig an den Seitenarmen 71, 72 befestigt. Zum Rahmengestell 60 gehört weiterhin die axiale Anordnung der Wellen 54, 56, 62. Dabei sind die verzahnten Wellen 54 und 62 endseitig fest mit den Seitenarmen 71 bzw. 72 verbunden. Dies kann dadurch erfolgen, daß die endseitige Verzahnung jeweils fest in ein Loch in den Seitenarmen 71 bzw. 72 eingepaßt ist. Dieses Rahmengestell 60 ist durch einen weiteren Schrittmotor 75 quer zu den Blendplatten 36, 38, d. h. in y-Richtung, verschiebbar. Der Motor 75 für die Querverschiebung ist in Fig. 6 neben dem Motor 66 gezeigt. Er ist dabei mit einer Einstellspindel 76, also mit einer Stange mit Gewinde 77, verbunden. Die Einstellspindel 76 ist durch ein Loch im zweiten Seitenarm 72 geführt und im ersten Seitenarm 71 drehbar gelagert. Sie dreht sich in einem Gewinde, das in einem Halteblock 78 quer zur Längsrichtung angebracht ist. Der Halteblock 78 erstreckt sich dabei mit seiner Längsrichtung parallel zur xz-Ebene. Aus Fig. 2, 5 und 6 ist die parallele Anordnung der Einstellspindel 76 zwischen den Führungsstangen 73 und 74 ersichtlich. Der Halteblock 78 ist endseitig abgerundet. Er nimmt ebenfalls die Führungsstangen 73, 74, ggf. in je einem Linear-Kugellager (vgl. Fig. 6), auf.

Der Halteblock 78 ist von einer Gleitachse 79 durchsetzt, und zwar parallel zur y-Richtung. Die Gleitachse 79 ist dabei durch eine (nicht gezeigte) Schraube am Halteblock 78 befestigt. Sie ist beidseitig durch Löcher in den Seitenarmen 71, 72 hindurchgeführt und kann in diesen gleiten oder rutschen (bei Betätigung der Spindel 76). Dreht sich die Einstellspindel 76 unter der Wirkung des Motors 75 in einer der beiden Richtungen, so wird das gesamte in sich starre Rahmengestell 60, bestehend aus den Bauteilen 54, 56, 57, 62, 71, 72, 73, 74 mit den Teilen 50, 51, 52 und 64, 65, 66 in einer Richtung parallel zur y-Achse verschoben. Der Halteblock 78 bleibt dabei stehen. Die Verschiebung erfolgt jeweils in ganzen Schritten der Blendplattenstärke d1, z. B. jeweils in ganzzahligen Vielfachen von d1 = 3 mm. Auf diese Weise wird die zu verstellende Blendplatte 36, 38 ausgewählt. Beim Verschieben werden übrigens beide Zahnräder 50, 64 gleichzeitig parallel zu sich selbst in derselben Richtung +y oder -y verschoben. Zur Verminderung der Reibung bei der Verschiebung in der Richtung parallel zur y-Achse sind im Rahmengestell 60 Linearkugellager 80 eingebaut, durch die die Achse 79 gleitet.

Die drei verzahnten Wellen 54, 56, 62 samt Antriebszahnrädern 50, 64 werden durch eine Druckeinrichtung an die Verzahnungen 43 sowie 30, 32 der Blendplatten 36, 38 bzw. der Außenplatten 26, 28 angedrückt. Diese Druckeinrichtung umfaßt ein Andruckstück 81, das beim Verschieben des Rahmengestells 60 in y-Richtung auf der oberen Fläche des Verbindungsteils 57 gleitet. Damit es beim Gleiten an den Schrauben 59, 69 nicht hängenbleibt, ist es an seiner unteren Seite mit einem Querschlitz 82 versehen.

Die Druckeinrichtung weist weiterhin einen Andruckbügel 83 auf. Dieser ist T-förmig und geschwungen ausgebildet. An dessen Fuß ist eine Einstellschraube 84 vorgesehen, die auf das Andruckstück 81 drückt. Die Einstellschraube 84 dient zum Einstellen des Anpreßdrucks der Wellen 54, 56, 62 gegen die Verzahnungen 30, 32, 43. Das Gegenlager wird durch die Einspannfläche zwichen dem Querarm des T-förmigen Andruckbügels 83 und der End- oder Oberfläche zweier Seitenbügel 87, 88 im Bereich zweier Verschraubungen 85, 86 gebildet.

Der T-förmige Andruckbügel 82 ist durch die Verschraubungen 85, 86 an den beiden beabstandeten, parallel ausgerichteten Seitenbügeln 87 bzw. 88 befestigt. Die beiden Seitenbügel 87, 88 sind jeweils L-förmig ausgebildet und mittels zweier Schrauben 89 bzw. 90 an den Außenplatten 26 bzw. 28 befestigt. Die Seitenbügel 87, 88 schließen somit auch die Blendplatten 36, 38 zwischen sich ein. Damit Platz für die y-Verschiebung zur Verfügung steht, ist der Abstand zwischen den Seitenbügeln 85, 86 um einiges kleiner als der Abstand zwischen den Seitenarmen 71, 72.

Das Andruckstück 81 könnte auch durch ein anderes Bauteil mit weniger Reibung ersetzt werden.

Soll eine der Blendplatten 36, 38 in x-Richtung verschoben werden, so wird folgendermaßen vorgegangen: Zunächst wird der Antriebsmotor 75 für die Querverstellung betätigt. Die resultierende seitliche Verstellung des Rahmengestells 60 in y-Richtung erfolgt in ganzen Schritten der Blendplattenstärke dl, beispielsweise jeweils um 3 mm. Auf diese Weise wird die zu verstellende Blendplatte 36, 38 ausgewählt, und zwar durch eins der beiden Antriebszahnräder 50, 64. Ist eine Blendplatte 36, 38 ausgewählt, so wird die Verstellung in x-Richtung vorgenommen. Es muß dabei betont werden, daß nur die ausgewählte Blendplatte 36, 38, also nur diejenige, die sich gerade im Eingriff mit dem Antriebszahnrad 50, 64 befindet, verstellt werden kann. Alle anderen Blendplatten 36, 38 sind durch die drei Wellen 54, 56, 62 arretiert. Die Verstellung der betreffenden Blendplatte 36, 38 erfolgt entweder über die erste Einstellwelle 51 durch den ersten Motor 52 oder über die zweite Einstellwelle 65 durch den Antriebsmotor 66. Diese Einstellwellen 51, 65 werden in ganzzahligen Zahnschritten gedreht, bis die betreffende Blendplatte 36, 38 die zuvor ausgewählte Stellung im Strahlprofil 24 erreicht hat.

Es soll noch angemerkt werden, daß bevorzugt eine Einrichtung zur Rückmeldung der Verschiebestellung der einzelnen Blendplatten 36, 38 vorgesehen sein kann. Eine solche (nicht gezeigte) Einrichtung kann einen Zähler umfassen, der bei jedem Verschieben die Zahl der betätigten Zähne der Verzahnung 43 der betreffenden Blendplatte 36, 38 zählt. Dabei kann es sich um einen mechanischen oder optischen Zähler handeln. Es kann sich aber auch um einen Zähler handeln, der die Schritte des gerade betätigten Schrittmotors 52 bzw. 66 zählt. Die Anzahl der zurückgelegten Schritte, die durch elektrische Impulse charakterisiert sind, ist dabei ein Maß für die Positionierung in x-Richtung.

Es war bereits dargelegt worden, daß die Wellen 54, 56, 62 als Arrettier- oder Bremselemente wirken, die alle - bis auf zweider Blendplatten 36, 38 festhalten. Stattdessen könnte auch ein anderes Halte- oder Arretierorgan gewählt werden, das in die Verzahnung 43 der Blendplatten 37 eingreift. Die Verzahnung 43 braucht dabei nicht unbedingt an einem Plattenrand zu liegen.

Nach Einstellen aller Blendplatten 36, 38 bleiben die Antriebszahnräder 50, 64 über den letzten zu verstellenden Blendplatten 36, 38 stehen. Diese werden durch den zugeordneten, unter Spannung stehenden Schrittmotor 52, 66 gehalten. Bei Ausfall der Antriebseinheit könnten sich nun die letzten Blendplatten, auf denen sich gerade die Antriebszahnräder befinden, aus ihrer Position bewegen. Dies wird vermieden, indem nach Einstellen der Blendplatten die Verriegelungseinrichtung 71 bis 76 noch um eine halbe Blendenplattenstärke weiter fährt. Damit sind alle Blendplatten 36, 38 mechanisch verriegelt.

Mittels des in den Fig. 2 bis 7 gezeigten Konturenkollimators läßt sich relativ rasch ein gewünschtes Strahlenprofil 24 einstellen. Auch beim Umkreisen des Konturenkollimators 4 während einer strahlentherapeutischen Behandlung muß nicht befürchtet werden, daß sich infolge ihres Gewichts einige der Blendplatten 36, 38, 36′, 38′ aus der vorgegebenen Positionierung lösen und damit die Strahlenkontur 24 verändern. Während einer solchen Umkreisung kann schrittweise in ausgewählten Umkreisungspositionen die Strahlenkontur neu eingestellt werden, um so ein optimales Strahlungsfeld zu applizieren.

Zusammenfassend läßt sich zu dem in den Figuren 2 bis 7 dargestellten Ausführungsbeispiel folgendes sagen: Zum Antrieb aller Blendplatten 36, 38, 36′, 38′ werden auf jeder Seite I, II des Konturenkollimators nur drei Schrittmotoren 52, 66, 75 benötigt, so daß zur Steuerung des gesamten Kollimatorsystems, unabhängig von der Blendplattenzahl, nur sechs Schrittmotoren 52, 66, 75 und 52′, 66′, 75′ benötigt werden.

Der Unterschied zu anderen technischen Lösungen der Einzelblattsteuerung besteht vorliegend darin, daß die einzelnen Blendplatten 36, 38 und 36′ , 38′ einer Seite I bzw. II nicht gleichzeitig, sondern nacheinander (seriell) angesteuert wer den. Der Antrieb erfolgt nach dem Zahnstangenprinzip, wobei ein Antriebszahnrad 50, 65 in Richtung der Antriebsachse 51, 65 schrittweise von Blendplatte zu Blendplatte 36, 38 zur Verstellung weitergestellt wird. Man könnte dies als "mechanisches Multiplexing" bezeichnen. Während der Steuerung einer Blendplatte sind die übrigen Blendplatten durch die Verriegelungsverzahnung (Zahnstangen 54, 56, 62) in ihren Positionen fixiert. Der Vorteil des seriellen Ansteuerungsprinzips liegt gegenüber Vielblattkollimatoren mit paralleler Ansteuerung in der wesentlich einfacheren Konstruktion, die geringeren Platz beansprucht und aufgrund des geringeren Gewichts auch als Nachrüstung an vorhandene Bestrahlungsgeräte eingesetzt werden kann. Auch asymmetrische Bestrahlungsfelder lassen sich relativ rasch und feinstufig einstellen.

Der Applikationsbereich irregulärer Bestrahlungsfelder betrifft praktisch nur Bestrahlungstechniken mit festen Einstrahlungsrichtungen. Hierbei spielt der Zeitbedarf von einigen Sekunden zur motorischen Profileinstellung keine Rolle. Darüber hinaus hat sich gezeigt, daß die mit kontinuierlichen Bewegungsstrahlungen erzielten Dosisverteilungen auch durch Bestrahlungen aus vielen festen Einstrahlungsrichtungen erreicht werden können. Dies ist mit dem vorliegenden Vielblattkollimator mit serieller Ansteuerung ohne weiteres möglich, so daß dieser Vielblattkollimator im gesamten Spektrum der strahlentherapeutischen Bestrahlungstechniken eingesetzt werden kann.

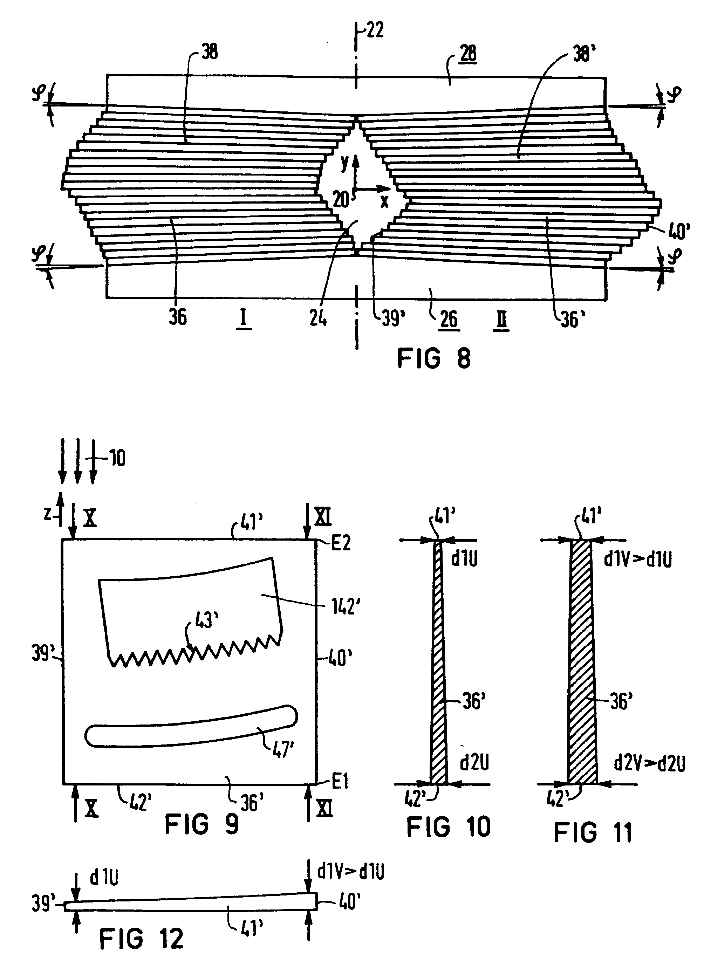

Nach Fig. 8 besteht der Konturenkollimator 4 wieder aus zwei Teilen oder Seiten I und II, die bezüglich einer vertikalen Ebene (y/z-Ebene) symmetrisch zueinander ausgebildet sind. Durch diese vertikale Ebene verläuft eine Symmetrielinie 20, die auf einer Mittellinie 22 senkrecht steht. Bei optimaler Justierung fällt die von einem Fokus ausgehende Hauptstrahlrichtung eines Strahlenbündels hochenergetischer Strahlung (z.B. Röntgenstrahlung) mit der Symmetrielinie zusammen. Die im Bereich der Symmetrielinie 20 liegende, durch Ausblendung er zielte Strahlenkontur (Profil) ist wieder mit 24 bezeichnet.

In einem gewissen Abstand voneinander sind eine erste und eine zweite vertikal gestellte, seitliche Außenplatte 26 bzw. 28 mit den Außenflächen parallel zueinander angeordnet. Das Besondere dieser Außenplatten 26, 28 besteht darin, daß ihre Innenflächen, und zwar ausgehend von der Mittellinie 22, jeweils unter einem vorgegebenen (kleinen) Winkel Phi zur Normalen (x-Richtung) dieser Mittellinie 22 ausgerichtet sind. Vorliegend ist dabei folgende Konstruktion gewählt: Ausgehend von der Mittellinie 22 verjüngen sich die beiden Außenplatten 26, 28 nach außen hin, d.h. in ± x-Richtung. Diese Verjüngung ist der Übersichtlichkeit wegen in Fig. 8 übertrieben dargestellt. Die Verjüngung ist in Wirklichkeit relativ gering, für die Funktion aber bedeutsam. Die beidseitig von der Mittellinie 22 einsetzende Verjüngung beträgt beispielsweise nur 0,54 mm (gemessen in y-Richtung) bei Fortschreiten um 10 cm nach außen (in +x-oder -x-Richtung). Das ergibt Phi gleich 5,4 x 10⁻³.

Der bei Fortschreiten in x-Richtung sich vergrößernde Abstand der beiden Innenflächen der Außenplatten 26, 28 gewährleistet, daß ein Klemmen beim Auffahren oder Öffnen der im folgenden beschriebenen Blendplatten 36, 38, 36′, 38′ nicht stattfinden kann.

Zwischen den beiden seitlichen Außenplatten 26, 28 ist wieder sowohl links als auch rechts ein Paket aus zwei Gruppen von gegeneinander verschieblichen Blendplatten 36, 38 bzw. 36′, 38′ angeordnet. Alle Blendplatten 36, 38, 36′, 38′ in der vorderen bzw. hinteren Gruppe sind auf beiden Seiten I und II in gleicher Weise aufgebaut und nebeneinanderliegend angeordnet. Das Paket 36, 38 kann ebenso wie das Paket 36′, 38′ beispielsweise 27 Blendplatten umfassen. Die Blendplatten 36, 38, 36′, 38′ bestehen aus einem gut verarbeitbaren Material, sind strahlungsresistent und in Strahlungsrichtung z gesehen strahlungsundurchlässig. Jede der Blendplatten kann beispielsweise eine Fläche von 10 cm x 10 cm aufweisen. Als Material kommt bevorzugt eine Wolfram-Nickel-Legierung zur Anwendung.

Aus Fig. 9 ist die Seitenansicht einer der Blendplatten 36′ zu entnehmen. Diese ist quadratisch ausgebildet und besitzt eine gekrümmte Führungsrille 47′, die zum Schwenken um den Fokus der Strahlenquelle dient. Eine im oberen Teil der Blendplatte 36′ untergebrachte Ausnehmung 142′ ist am unteren Rand mit einer Verzahnung 43′ versehen, die mit einem (nicht gezeigten) Stellorgan zum feinfühligen Schwenken oder Verschieben der Blendplatte 36′ im Eingriff steht.

Es war bereits erwähnt worden, daß die Innenflächen der Außenplatten 26, 28 nach außen (in x-Richtung) voneinander zurückweichen. Damit kann, wie ebenfalls bereits dargelegt, ein Klemmen der Blendplatten 36, 38, 36′, 38′ beim Heraus fahren verhindert werden. Würden nun aber planparallele Blendplatten verwendet werden, so könnte sich beim Rück- oder Zufahren in Richtung in Richtung auf die Mittellinie 22 ein Wackeln der Blendplatten ergeben. Damit würden zwischen den Blendplatten Zwischenräume geschaffen, durch die Strahlung hindurchtreten könnte. Dies muß aber unter allen Umständen verhindert werden. Um dieses zu gewährleisten, sind alle Blendplatten 36, 38, 36′, 38′ in Richtung auf die Mittellinie 22 leicht keilförmig geschliffen. Dies wird im folgenden anhand der Fig. 9 bis 12 verdeutlicht.

Aus den Fig. 9 bis 12 ergibt sich, daß die Blendplatte 36′ an der der Mittellinie 22 der Strahlung 10 zugewandten Kante 39′ eine Dicke d1U, d2U (vgl. Schnitt X-X durch den linken Teil der Blendplatte 36′) und an der von der Mittellinie 22 der Strahlung 10 abgewandten Kante 40′ eine Dicke d1V, d2V (vgl. Schnitt XI-XI durch den rechten Teil der Blendplatte 36′) aufweist. Es gilt, daß d1V größer ist als d1U; ebenso ist d2V größer als d2U. Dies wird noch einmal bei einem Blick auf Fig. 12, d.h. auf die der Strahlung zugewandte Kante 41′ der Blendplatte 36′, deutlich.

Der Keilwinkel, der sich beim Fortschreiten von der Kante 39′ zur Kante 40′ ergibt, liegt im Bereich von 1/100 mm oder etwas mehr, insbesondere bei 4/100 mm, bei einer Blendplatte 36′ einer Breite von 10 cm. Eine ausreichende Maßgenauigkeit kann bei der Bearbeitung eines Materials wie der voranstehend erwähnten Wolfram-Nickel-Legierung gewährleistet werden.

Als zusätzliche Maßnahme ist vorgesehen, daß die Blendplatten 36, 38, 36′, 38′ an der der Strahlung 10 zugewandten Kante 41′ eine Dicke d1U, d1V aufweisen, die kleiner ist als die Dicke d2U, d2V an der von der Strahlung 10 abgewandten Kante 42′. Man kann diese beiden Tatsachen auch so ausdrücken: Die Blendplatte 36′ ist in zwei aufeinander senkrechten Richtungen, die vorgegeben sind durch die Kanten 41′, 42′ und 39′, 40′, keilförmig geschliffen.

Zusammenfassend läßt sich also folgendes sagen: Aus fertigungstechnischen Gründen können Blendplatten häufig nicht ideal in Richtung des Fokus verjüngt geschliffen werden. Um den geometrischen Fehler möglichst gering zu halten, werden die Blendplatten 36, 38, 36′, 38′ (bezogen auf den Radius der Führungsrille 47) vorliegend zum Radiusmittelpunkt hin verjüngt geschliffen.

Man kann dies auch so ausdrücken: Da die Blendplatten 36, 38, 36′, 38′ mit ihrer von der Mittellinie 22 abgewandten Seite (z.B. 40′ in Fig. 9) während des Verfahrens in der Führungsrille 47 in x-Richtung zum Fokus einen Radius beschreiben, steigt z.B. die untere Ecke E1 (vgl. Fig. 9) der verfahrenden, nach oben sich verjüngenden Blendplatten 36′ gegenüber den restlichen Blendplatten 36′ und den feststehenden Außenplatten 26, 28 nach oben. Wenn nicht genügend Spiel vorhanden ist - was wegen der Gefahr des Strahlendurchsatzes unerwünscht ist - kann es bei den früher beschriebenen Blendplatten zur Klemmung kommen. Um dies zu vermeiden, sind vorliegend die Außenplatten 26, 28 um die Dickendifferenz der einzelnen, nach oben ansteigenden Blendplatten 36, 38, 36′, 38′, multipliziert mit der Anzahl der Blendplatten 36, 38, 36′ bzw. 38′, nach außen freigeschliffen (kleiner Winkel Phi, vgl. Fig. 8).

## Patentansprüche

1. Konturenkollimator für die Strahlentherapie mit einer vorgegebenen Anzahl von gegeneinander verschieblich angeordneten Blendplatten (36, 38, 36′, 38′), die zwischen zwei Außenplatten (26, 28) angeordnet und mit einer Kante (39, 39′ ) von zwei Seiten an eine Mittellinie (22) heranschiebbar sind, durch die eine Symmetrielinie (20) der Strahlung (10) verläuft, zur Bildung einer Strahlenkontur (24), wobei die Innenflächen der Außenplatten (26, 28) unter vorgegebenem Winkel (Phi) zur Normalen (x) der Mittellinie (22) ausgerichtet sind, wobei die Blendplatten (36, 38, 36′, 38′) an der der Mittellinie (22) der Strahlung (10) zugewandten Kante (39, 39′) eine geringere Dicke (d1U, d2U) aufweisen als an der von der Mittellinie (22) der Strahlung (10) abgewandten Kante (40′) (Fig. 8 bis 12) und wobei die Blendplatten (36, 38, 36′, 38′) an der der Strahlungsquelle (10) zugewandten Kante (41′) eine kleinere Dicke (d1U, d1V) aufweisen als an der von der Strahlungsquelle (10) abgewandten Kante (42′).

2. Konturenkollimator nach Anspruch 1, **dadurch gekennzeichnet,** daß die Blendplatten (36, 38, 36′, 38′ ) in zwei aufeinander senkrechten Richtungen keilförmig geschliffen sind.

3. Konturenkollimator nach Anspruch 1 oder 2, **dadurch gekennzeichnet**, daß die Außenplatten (26, 28) - von der Mittellinie (22) aus - sich nach außen (x-Richtung) verjüngen.

4. Konturenkollimator nach Anspruch 3, **dadurch gekennzeichnet**, daß sich bei einer Fokusentfernung der Strahlenquelle von den oberen Kanten (41′) der Blendplatten (36, 38, 36′, 38′) von etwa 46 cm die Außenplatten (26, 28) auf jeweils 10 cm etwa 0,54 mm verjüngen.

5. Konturenkollimator nach Anspruch 3 oder 4, **dadurch** **gekennzeichnet**, daß die Außenplatten (26, 28) sich beidseitig von der Mittellinie (22) aus nach außen verjüngen.

6. Konturenkollimator nach einem der Ansprüche 1 bis 5, **dadurch gekennzeichnet**, daß er bezüglich einer Mittellinie (22) symmetrisch aufgebaut ist.

7. Konturenkollimator nach einem der Ansprüche 1 bis 6, **dadurch gekennzeichnet**, daß eine Verzahnung (43) an einem Rand der Blendplatten (36, 38, 36′, 38′; 37) angeordnet ist.

8. Konturenkollimator nach einem der Ansprüche 1 bis 7, **dadurch gekennzeichnet**, daß die Blendplatten (36, 38, 36′, 38′; 37) um einen gemeinsamen Fokus (F) schwenkbar sind.

9. Konturenkollimator nach Anspruch 8, **dadurch gekennzeichnet**, daß die Blendplatten (37, 37′) jeweils mit einer Führungsrille (47, 47′ ) versehen sind.

10. Konturenkollimator nach Anspruch 9, **dadurch gekennzeichnet**, daß die Führungsrille (47, 47′ ) gekrümmt ist.

11. Konturenkollimator nach Anspruch 9 oder 10, **dadurch gekennzeichnet**, daß zur Führung der Blendplatten (37, 37′ ) mittels der Führungsrillen (47, 47′ ) zwei parallel zueinander angeordnete Führungsbolzen (48, 49, 48′, 49′ ) vorgesehen sind.

12. Konturenkollimator nach einem der Ansprüche 1 bis 11, **dadurch gekennzeichnet**, daß die Blendplatten (36, 38, 36′, 38′; 37) aus einem Wolfram enthaltenden Material bestehen.

13. Konturenkollimator nach einem der Ansprüche 1 bis 12, **dadurch gekennzeichnet**, daß die Blendplatten (36) und die zusätzlichen Blendplatten (38) jeweils über die Mittellinie (22) hinaus in die andere Seite (I, II) verschiebbar sind.

## Claims

1. Contour collimator for radiation therapy, having a predetermined number of diaphragm plates (36, 38, 36′, 38′), which are arranged displaceably with respect to one another, are arranged between two outer plates (26, 28) and can be pushed with one edge (39′, 39′) from two sides onto a centre line (22), through which a line of symmetry (20) of the radiation (10) extends, to form a radiation contour (24), the inner surfaces of the outer plates (26, 28) being aligned at a predetermined angle (Phi) to the normal (x) of the centre line (22), the diaphragm plates (36, 38, 36′, 38′) having a lower thickness (d1U, d2U) on the edge (39, 39′) facing the centre line (22) of the radiation (10) than on the edge (40′) facing away from the centre line (22) of the radiation (10) (Figs. 8 to 12) and the diaphragm plates (36, 38, 36′, 38′) having a smaller thickness (d1U, d1V) on the edge (41′) facing the radiation source (10) than on the edge (42′) facing away from the radiation source (10).

2. Contour collimator according to Claim 1, characterized in that the diaphragm plates (36, 38, 36′, 38′) are ground in a wedge-shape in two directions perpendicular to each other.

3. Contour collimator according to Claim 1 or 2, characterized in that the outer plates (26, 28) taper outwards (x-direction) from the centre line (22).

4. Contour collimator according to Claim 3, characterized in that, at a distance of the focus of the radiation source from the upper edges (41′) of the diaphragm plates (36, 38, 36′, 38′) of about 46 cm, the outer plates (26, 28) taper by about 0.54 mm over each 10 cm.

5. Contour collimator according to Claim 3 or 4, characterized in that the outer plates (26, 28) taper outwards on both sides from the centre line (22).

6. Contour collimator according to one of Claims 1 to 5, characterized in that it is constructed symmetrically with respect to a centre line (22).

7. Contour collimator according to one of Claims 1 to 6, characterized in that a toothing (43) is arranged on one edge of the diaphragm plates (36, 38, 36′, 38′; 37).

8. Contour collimator according to one of Claims 1 to 7, characterized in that the diaphragm, plates (36, 38, 36′, 38′; 37) can be pivoted about a common focus (F).

9. Contour collimator according to Claim 8, characterized in that the diaphragm plates (37,37′) are in each case provided with a guide groove (47, 47′).

10. Contour collimator according to Claim 9, characterized in that the guide groove (47, 47′) is curved.

11. Contour collimator according to Claim 9 or 10, characterized in that, to guide the diaphragm plates (37, 37′) by means of the guide grooves (47, 47′), two guide pins (48, 49, 48′, 49′) are provided, arranged parallel to each other.

12. Contour collimator according to one of Claims 1 to 11, characterized in that the diaphragm plates (36, 38, 36′, 38′; 37) comprise a tungsten-containing material.

13. Contour collimator according to one of Claims 1 to 12, characterized in that the diaphragm plates (36) and the additional diaphragm plates (38) are in each case displaceable beyond the centre line (22) into the other side (I, II).

## Revendications

1. Collimateur de contours pour thérapie par irradiation avec un nombre prédéterminé de plaques d'occultation (33, 38, 36′, 38′) disposées mobiles les unes par rapport aux autres, entre deux plaques extérieures (26, 28) et déplaçables par une arête (39, 39′) depuis deux côtés pour se situer sur une ligne médiane (22), par laquelle passe une ligne de symétrie (20) du rayonnement (10), pour constituer un contour de rayon (24), les faces intérieures des plaques extérieures (26, 28) étant orientées avec un angle (Phi) prédéterminé par rapport à la normale (x) de la ligne médiane (22), les plaques d'occultation (36, 38, 36′, 38′) présentant sur l'arête (39, 39′) tournée vers la ligne médiane (22) du rayonnement (10) une épaisseur (d1U, d2U) moindre que sur l'arête (40′) opposée à la ligne médiane (22) du rayonnement (10) (figure 8 à 12), et les plaques d'occultation (36, 38, 36′, 38′) présentant sur l'arête (41′) tournée vers la source de rayonnement (10) une épaisseur (D1U, D1V) moindre que sur l'arête (42′) opposée à la source de rayonnement (10).

2. Collimateur de contours selon la revendication 1, caractérisé en ce que les plaques d'occultation (36, 38, 36′ 38′) sont meulées à une configuration cunéiforme dans deux directions perpendiculaires l'une à l'autre.

3. Collimateur de contours selon la revendication 1 ou 2, caractérisé en ce que les plaques extérieures (26, 28) vont en s'effilant, depuis la ligne médiane (22), en allant dans la direction de l'extérieur (direction x).

4. Collimateur de contours selon la revendication 3, caractérisé en ce que, dans la cas d'une distance focale entre la source de rayonnement et les arêtes supérieures (41′) des plaques d'occultation (36, 38, 36′, 38′) d'à peu près 46 cm, les plaques extérieures (26, 28) de dimension 10 cm vont chacune en s'effilant, d'à peu près 0,54 mm.

5. Collimateur de contours selon la revendication 3 ou 4, caractérisé en ce que les plaques extérieures (26, 28) vont en s'effilant des deux cotés de la ligne médiane (22), en direction de l'extérieure.

6. Collimateur de contours selon l'une des revendications 1 à 5, caractérisé en ce que sa structure est symétrique par rapport à une ligne médiane (22).

7. Collimateur de contours selon l'une des revendications 1 à 6, caractérisé en ce qu'une denture (43) est disposée sur un bord des plaques d'occultation (36, 38, 36′, 38′; 37).

8. Collimateur de contours selon l'une des revendications 1 à 7, caractérisé en ce que les plaques d'occultation (36, 38, 36′, 38′, 37) sont susceptibles de pivoter autour d'un foyer (F) commun.

9. Collimateur de contours selon la revendication 8, caractérisé en ce que les plaques d'occultation (37, 37′) sont chacune pourvues d'une rainure de guidage (47, 47′).

10. Collimateur de contours selon la revendication 9, caractérisé en ce que la rainure de guidage (47, 47′) est incurvée.

11. Collimateur de contours selon la revendication 9 ou 10, caractérisé en ce que deux boulons de guidage (48, 49, 48′, 49′) sont prévus, disposés parallèlement l'un à l'autre, pour assurer le guidage des plaques d'occultation (37, 37′) au moyen des rainures de guidage (47, 47′).

12. Collimateur de contours selon l'une des revendications 1 à 11, caractérisé en ce que les plaques d'occultation (36, 38′, 36′, 38′; 37) sont constituées d'un matériau contenant du tungstène.

13. Collimateur de contours selon l'une des revendications 1 à 12, caractérisé en ce que les plaques d'occultation (36) et- les plaques d'occultation (38) supplémentaires sont chacune déplaçables en franchissant la ligne médiane (22), pour passer de l'autre coté (I, II).
